# EUROPEAN PATENT SPECIFICATION

(11) **EP 3 254 301 B1**
(45) Date of publication and mention of the grant of the patent: **31.03.2021**
(21) Application number: 16746213.4
(22) Date of filing: 05.02.2016
(51) Int. Cl.: H01J 49/00, G01N 33/92

(54) **LIPID SCREENING PLATFORM ALLOWING A COMPLETE SOLUTION FOR LIPIDOMICS RESEARCH**
LIPID-SCREENING-PLATTFORM MIT ERMÖGLICHUNG EINER KOMPLETTEN LÖSUNG ZUR LIPIDOMIKFORSCHUNG
PLATE-FORME DE CRIBLAGE DE LIPIDES PERMETTANT UNE SOLUTION COMPLÈTE POUR LA RECHERCHE LIPIDOMIQUE

(30) Priority: 06.02.2015 US 201562113290 P
(43) Date of publication of application: 13.12.2017
(73) Proprietor: DH Technologies Development Pte. Ltd., Singapore 739256 (SG)
(72) Inventor: UBHI, Baljit K., San Mateo, California 94402 (US); CONNOR, Alexandria, West Sacramento, California 95691 (US); DUCHOSLAV, Eva, Toronto, Ontario M2J 2N3 (CA); WANG, Leo, San Jose, California 95131 (US); BAKER, Paul, Pittsburgh, Pennsylvania 15208 (US); WATKINS, Steven, Davis, California 95618 (US)
(74) Representative: J A Kemp LLP
(86) International application number: PCT/IB2016/050613
(87) International publication number: WO 2016/125120

(56) References cited:
- WO-A1-2013/184995
- WO-A1-2014/102577
- US-A1- 2006 151 691
- US-A1- 2009 299 671
- US-A1- 2011 127 419
- Volker Kruft: "Lipidomics: Lipidyzer (TM) as a complete solution for the quantitation of more than 1000 lipids", , 1 January 2015 (2015-01-01), XP055504632, Retrieved from the Internet: URL:http://congress.fedlab.ru/upload/congr ess/zal%20PipoGoB/Kruft%20lipidyzer.pdf [retrieved on 2018-09-05]
- TUULIA P. I. LINTONEN ET AL: "Differential Mobility Spectrometry-Driven Shotgun Lipidomics", ANALYTICAL CHEMISTRY, vol. 86, no. 19, 26 August 2014 (2014-08-26), pages 9662-9669, XP055504649, US ISSN: 0003-2700, DOI: 10.1021/ac5021744
- ALEXANDRE A. SHVARTSBURG ET AL: "Separation and Classification of Lipids Using Differential Ion Mobility Spectrometry", JOURNAL OF THE AMERICAN SOCIETY FOR MASS SPECTROMETRY., vol. 22, no. 7, 12 April 2011 (2011-04-12) , pages 1146-1155, XP055288182, US ISSN: 1044-0305, DOI: 10.1007/s13361-011-0114-z
- Anonymous: "Analyst (R) 1.6.2 Software - SelexION (TM) Technology User Guide", , 1 May 2013 (2013-05-01), XP055504655, Retrieved from the Internet: URL:https://sciex.com/Documents/tech%20not es/selexion-technology-user-guide-eng.pdf [retrieved on 2018-09-05]
- Baljit K Uhbi ET AL: "A Novel Lipid Screening Platform that Provides a Complete Solution for Lipidomics Research", , 1 January 2015 (2015-01-01), XP055504630, Retrieved from the Internet: URL:https://cdn.technologynetworks.com/TN/ Resources/PDF/The_Lipidyzer_Platform%20Tec h%20Note%20RUO-MKT-02-2871-A.pdf [retrieved on 2018-09-05]
- RAY JULIE A ET AL: "Performance enhancement in the measurement of 5 endogenous steroids by LC-MS/MS combined with differential ion mobility spectrom", CLINICA CHIMICA ACTA, ELSEVIER BV, AMSTERDAM, NL, vol. 438, 9 August 2014 (2014-08-09), pages 330-336, XP029020346, ISSN: 0009-8981, DOI: 10.1016/J.CCA.2014.07.036

## Description

### INTRODUCTION

The teachings herein relate to high-field asymmetric waveform ion mass spectrometry (FAIMS) or differential mobility spectrometry (DMS). More particularly the teachings herein relate to systems and methods for quantitating lipids using a FAIMS or DMS device.

### Background

Lipids are naturally occurring organic molecules. They are generally characterized as being insoluble in water and soluble by organic solvents. Lipids are abundant in many different types of animal samples or matrices including, but not limited to, plasma, heart, liver, brain, muscle, whole blood, urine and cells. Lipids are also abundant in other types of samples or matrices including, but not limited to, milk, edible oils, and meat. Identifying and quantifying the lipids in an animal matrix can be useful in identifying and treating disease, for example. Identifying and quantifying the lipids in other matrices, such as food matrices, can be useful in verifying the integrity or safety of those matrices. Unfortunately, currently, there is no single system or method for identifying and quantifying all the lipids present in a particular matrix in a reasonable amount of time.

Tandem mass spectrometry is a method that is used to both identify and quantify or quantitate compounds or molecules in a sample matrix. In a tandem mass spectrometer, a precursor ion is selected in a mass filter, fragmented, and the product ions are analyzed in a mass analyzer. The entire process of selecting a precursor ion, fragmenting it, and analyzing the resulting product ions by mass-to-charge ratio (m/z) is referred to as tandem mass spectrometry, mass spectrometry/mass spectrometry (MS/MS), or an MS/MS scan. The product ion intensity or spectrum produced from MS/MS can be used to identify a compound (the precursor ion) in a sample, and the intensity of one or more product ions can be used to quantitate the amount of the compound present in the sample.

Information dependent analysis (IDA) is a flexible tandem mass spectrometry method in which a user can specify criteria for performing MS/MS while a sample is being introduced into the tandem mass spectrometer. For example, in an IDA method a precursor ion or mass spectrometry (MS) survey scan is performed to generate a precursor ion peak list. The user can select criteria to filter the peak list for a subset of the precursor ions on the peak list. MS/MS is then performed on each precursor ion of the subset of precursor ions. A product ion intensity or spectrum is produced for each precursor ion. MS/MS is repeatedly performed on the precursor ions of the subset of precursor ions as the sample is being introduced into the tandem mass spectrometer. The sample is introduced to the ion source of the tandem mass spectrometer through an injection or a chromatographic run, for example.

One type of MS/MS performed during an IDA method is referred to as multiple reaction monitoring (MRM), selected reaction monitoring (SRM), or as an MRM or SRM scan or transition. In an MRM scan, for example, a single precursor ion is selected and fragmented, a single product ion is then selected from the resulting product ions, and the selected product ion is mass analyzed. MRM is typically used for quantitative analysis. In other words, MRM is typically used to quantitate the amount of a precursor ion in a sample from the intensity of a product ion.

Some tandem mass spectrometers, such as AB SCIEX's QTRAP®, allow IDA methods to perform MRM. This is very useful for multi-analyte screening methods, including lipid analysis.

Unfortunately, however, even using MRM based tandem mass spectrometry methods it has been difficult to characterize all of the lipids in a particular type of matrix. Complete lipid characterization has been difficult because many matrices include large numbers of isobaric and near isobaric interferences that confound identification and accurate quantitation. In general, isobaric interference occurs when a sample contains another compound that has or produces a product ion with a similar m/z as the analyte, or compound of interest. In the case of lipids, many classes of lipids include lipids that interfere with the lipids of another class. This problem, coupled with complicated sample preparation techniques and data analysis needed for lipids, highlights the need for a complete solution that addresses these difficulties and provides a simplified method for analysis.

As a result, system and methods are need to identify and quantitate all the lipids present in a particular type of sample matrix with a reasonable amount of time.

"Lipidomics: Lipidyzer (TM) as a complete solution for the quantitation of more than 1000 lipids", 2015, by Volker Kruft and available on http:// congress.fedlab.ru/upload/congress/3a 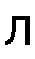 %20 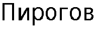 /Kruft%20lipidyzer.pdf, discloses a lipidomics analysis system comprising a DMS and MS/MS portion.

"TUULIA P. I. LINTONEN ET AL: "Differential Mobility Spectrometry-Driven Shotgun Lipidomics", ANALYTICAL CHEMISTRY, vol. 86, no. 19, 26 August 2014, pages 9662-9669, discloses DMS methods for separating isobaric and isometic lipids.

### SUMMARY

The invention provides a system according to claim 1 and a method according to claim 11.

Various embodiments of systems and methods are provided for quantitating known lipid molecules of a particular matrix using a differential mobility spectrometry (DMS) device during a targeted multiple reaction monitoring (MRM) acquisition experiment. Before the experiment, the known lipid molecules of the matrix are grouped into lipid classes and the lipid classes are further grouped into a pass-through group and a mobility separation group. The pass-through group includes lipid classes without lipid molecules known to produce isobaric interferences with other lipid molecules. The mobility separation group includes lipid classes with lipid molecules known to produce isobaric interferences.

An HPLC separation system introduces a matrix sample via infusion through low-carryover tubing. An ion source ionizes the matrix sample producing an ion beam. A DMS device receives an ion beam from the ion source for a first injection and a second injection of the matrix sample. For the first injection, the DMS device is put into passive mode and does not actively resolve lipid molecules. For the second injection, the DMS device is used to resolve isobaric interferences. In order to resolve isobaric interferences, the DMS device receives and applies different compensation voltages (CoV) values to the ion beam. The DMS device receives and applies CoV values stored for each of the lipid classes in the mobility separation group.

A tandem mass spectrometer receives a first ion beam from the DMS device for the first injection. The tandem mass spectrometer performs, for a first plurality of cycles, an MRM scan for at least one MRM transition for each lipid molecule of each lipid class of the pass-through group and stores a first set of intensity values of each MRM scan for the first plurality of cycles.

The tandem mass spectrometer receives a second ion beam that is separated according to ion mobility by the DMS device for the second injection. The tandem mass spectrometer performs, for a second plurality of cycles, an MRM scan for at least one MRM transition for each lipid molecule of each lipid class of the mobility separation group and stores a second set of intensity values of each MRM scan for the second plurality of cycles.

A processor in communication with the mass spectrometer and the DMS applies quantitates each lipid molecule in the matrix sample by comparing the first set of intensity values and the second set of intensity values to MRM intensity and concentration values for the known standards of the known lipid molecules of the matrix.

### BRIEF DESCRIPTION OF THE DRAWINGS

The skilled artisan will understand that the drawings, described below, are for illustration purposes only. The drawings are not intended to limit the scope of the present teachings in any way.
Figure 1 is a block diagram that illustrates a computer system, upon which embodiments of the present teachings may be implemented.
Figure 2 is a schematic diagram of an exemplary differential mobility spectrometry (DMS) device.
Figure 3 is a diagram showing an initial hierarchy of a multiple reaction monitoring (MRM) method for identifying and quantitating lipids in a particular matrix, in accordance with various embodiments.
Figure 4 is a diagram showing a hierarchy after grouping by isobaric interferences of an MRM method for identifying and quantitating lipids in a particular matrix, in accordance with various embodiments.
Figure 5 is a schematic diagram showing a system for quantitating lipids of a matrix sample using a DMS device during a targeted MRM acquisition experiment, in accordance with various embodiments.
Figure 6 is a flowchart showing a method for quantitating lipids of a matrix sample using a DMS device during a targeted MRM acquisition experiment, in accordance with various embodiments.

Before one or more embodiments of the present teachings are described in detail, one skilled in the art will appreciate that the present teachings are not limited in their application to the details of construction, the arrangements of components, and the arrangement of steps set forth in the following detailed description or illustrated in the drawings. Also, it is to be understood that the phraseology and terminology used herein is for the purpose of description and should not be regarded as limiting.

### DESCRIPTION OF VARIOUS EMBODIMENTS

### COMPUTER-IMPLEMENTED SYSTEM

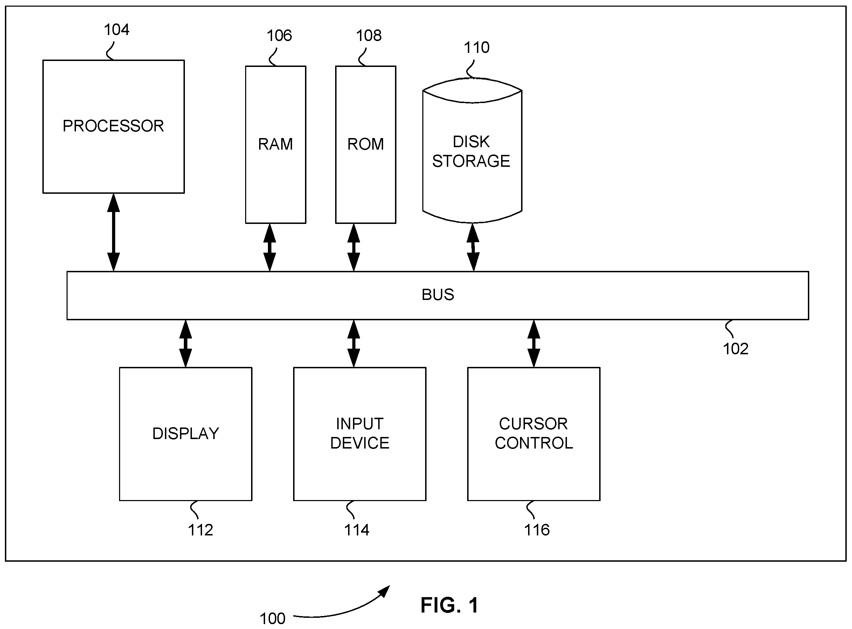

Figure 1 is a block diagram that illustrates a computer system 100, upon which embodiments of the present teachings may be implemented. Computer system 100 includes a bus 102 or other communication mechanism for communicating information, and a processor 104 coupled with bus 102 for processing information. Computer system 100 also includes a memory 106, which can be a random access memory (RAM) or other dynamic storage device, coupled to bus 102 for storing instructions to be executed by processor 104. Memory 106 also may be used for storing temporary variables or other intermediate information during execution of instructions to be executed by processor 104. Computer system 100 further includes a read only memory (ROM) 108 or other static storage device coupled to bus 102 for storing static information and instructions for processor 104. A storage device 110, such as a magnetic disk or optical disk, is provided and coupled to bus 102 for storing information and instructions.

Computer system 100 may be coupled via bus 102 to a display 112, such as a cathode ray tube (CRT) or liquid crystal display (LCD), for displaying information to a computer user. An input device 114, including alphanumeric and other keys, is coupled to bus 102 for communicating information and command selections to processor 104. Another type of user input device is cursor control 116, such as a mouse, a trackball or cursor direction keys for communicating direction information and command selections to processor 104 and for controlling cursor movement on display 112. This input device typically has two degrees of freedom in two axes, a first axis (*i.e*., x) and a second axis (*i.e.,* y), that allows the device to specify positions in a plane.

A computer system 100 can perform the present teachings. Consistent with certain implementations of the present teachings, results are provided by computer system 100 in response to processor 104 executing one or more sequences of one or more instructions contained in memory 106. Such instructions may be read into memory 106 from another computer-readable medium, such as storage device 110. Execution of the sequences of instructions contained in memory 106 causes processor 104 to perform the process described herein. Alternatively hard-wired circuitry may be used in place of or in combination with software instructions to implement the present teachings. Thus implementations of the present teachings are not limited to any specific combination of hardware circuitry and software.

The term "computer-readable medium" as used herein refers to any media that participates in providing instructions to processor 104 for execution. Such a medium may take many forms, including but not limited to, non-volatile media, volatile media, and transmission media. Non-volatile media includes, for example, optical or magnetic disks, such as storage device 110. Volatile media includes dynamic memory, such as memory 106. Transmission media includes coaxial cables, copper wire, and fiber optics, including the wires that comprise bus 102.

Common forms of computer-readable media include, for example, a floppy disk, a flexible disk, hard disk, magnetic tape, or any other magnetic medium, a CD-ROM, digital video disc (DVD), a Blu-ray Disc, any other optical medium, a thumb drive, a memory card, a RAM, PROM, and EPROM, a FLASH-EPROM, any other memory chip or cartridge, or any other tangible medium from which a computer can read.

Various forms of computer readable media may be involved in carrying one or more sequences of one or more instructions to processor 104 for execution. For example, the instructions may initially be carried on the magnetic disk of a remote computer. The remote computer can load the instructions into its dynamic memory and send the instructions over a telephone line using a modem. A modem local to computer system 100 can receive the data on the telephone line and use an infra-red transmitter to convert the data to an infra-red signal. An infra-red detector coupled to bus 102 can receive the data carried in the infra-red signal and place the data on bus 102. Bus 102 carries the data to memory 106, from which processor 104 retrieves and executes the instructions. The instructions received by memory 106 may optionally be stored on storage device 110 either before or after execution by processor 104.

Instructions configured to be executed by a processor to perform a method can be stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

The following descriptions of various implementations of the present teachings have been presented for purposes of illustration and description. It is not exhaustive and does not limit the present teachings to the precise form disclosed. Modifications and variations are possible in light of the above teachings or may be acquired from practicing of the present teachings. Additionally, the described implementation includes software but the present teachings may be implemented as a combination of hardware and software or in hardware alone. The present teachings may be implemented with both object-oriented and non-object-oriented programming systems.

### LIPID SCREENING PLATFORM

As described above, a major challenge in lipid analysis is the many isobaric and near isobaric interferences present in highly complex samples that confound identification and accurate quantitation. This problem, coupled with complicated sample preparation techniques and data analysis, highlights the need for a complete solution that addresses these difficulties and provides a simplified method for analysis.

In various embodiments, a novel lipidomics platform is developed that includes simplified sample preparation, automated methods, and streamlined data processing techniques that enables facile, quantitative lipid analysis. Differential mobility spectrometry (DMS) has been shown to be able to resolve isobaric interferences.

For IDA methods, differential mobility spectrometry (DMS) has been shown to be able to resolve isobaric interferences. Specifically, SCIEX's SelexION™ ion mobility technology has been utilized to enhance the quality of m/z analysis by pre-separating ions of similar m/z, thereby removing these isobaric interferences.

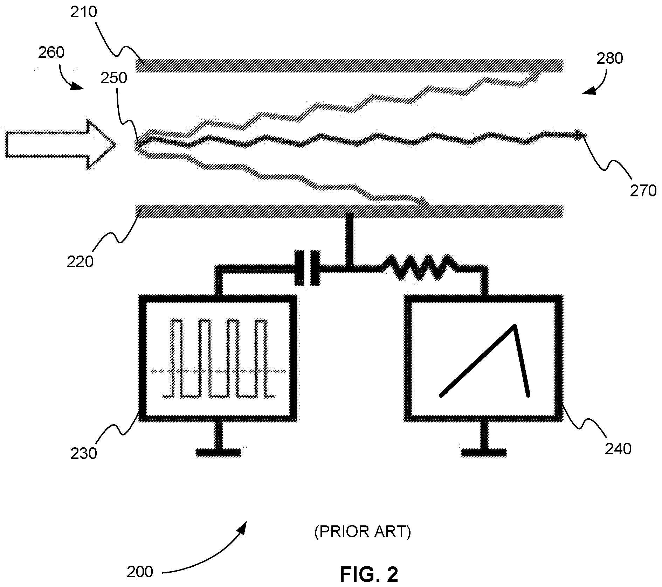

Figure 2 is a schematic diagram of an exemplary DMS device 200. DMS device 200 includes two parallel flat plates, plate 210 and plate 220. Radio frequency (RF) voltage source 230 applies an RF separation voltage (SV) across plate 210 and plate 220, and direct current (DC) voltage source 240 applies a DC compensation voltage (CoV) across plate 210 and plate 220. Ions 250 enter DMS device 200 in a transport gas at opening 260. The separation of ions 250 in DMS device 200 is based upon differences in their migration rates under high versus low electric fields.

Unlike traditional ion mobility, ions 250 are not separated in time as they traverse the device. Instead, ions 250 are separated in trajectory based on the difference in their mobility between the high field and low field portions of applied RF voltage source 230. The high field is applied between plate 210 and plate 220 for a short period of time, and then a low field is applied in the opposite direction for a longer period of time. Any difference between the low-field and high-field mobility of an ion of a compound of interest causes it to migrate towards one of the plates. The ion is steered back towards the center-line of the device by the application of a second voltage offset, known as the CoV of DC voltage source 240, a compound-specific parameter that can be used to selectively filter out all other ions. Rapid switching of the CoV allows the user to concurrently monitor many different compounds. Ions 270 selected by the combination of SV and CoV, leave DMS device 200 through opening 280 to the remainder of the mass spectrometer (not shown). DMS device 200 is located between an ion source (not shown) and the remainder of the mass spectrometer, for example.

In general, DMS device 200 has two modes of operation. In the first mode, DMS device 200 is on, SV and CoV voltages are applied, and ions are separated. This is, for example, the enabled mode.

In the second mode of operation, DMS device 200 is off, the SV is set to zero and ions 250 are simply transported from opening 260 to opening 280. This is, for example, the disabled or transparent mode of DMS device 200.

In the enabled mode, DMS device 200 can acquire data for a single MRM transition in 25 milliseconds (ms), for example, including an inter-scan pause time of 20 ms. In transparent mode, the delay through DMS device 200 is negligible.

In various embodiments, a DMS device is used for targeted profiling of hundreds of lipid species from 10 different lipid classes allowing for comprehensive coverage.

### Experimental Method

Serum samples were analyzed quantitatively using a unique internal standard labeling protocol, a novel selectivity tool (DMS) and novel lipid data analysis software. Applying a kit for simplified sample extraction and preparation, a serum matrix is used following the protocols provided. An LC-DMS-QTRAP® System is used, for example, for targeted profiling of hundreds of lipid species from 10 different lipid classes, allowing for comprehensive coverage. This system allows for: 1. quantitative results for each lipid class as a sum of individual species; 2. mole percent composition, obtained computationally from lipid molecular species data; and 3. accurate lipid species compositions. The data is compared with historical data generated by alternative methods. Samples are quantitated using software accompanying the full solution, which incorporates the novel labeled internal standards available as a kit, developed for this platform.

Quantitative lipid species measurements are obtained from the following complex lipid classes: diacylglycerols (DAGs), triacylglycerols (TAGs), phosphatidylcholines (PCs), phosphatidylethanolamines (PEs), sphingomyelins (SMs), lysophosphatidylcholines (LPCs) and lysophosphatidylethanolamines (LPEs), free fatty acids (FFAs), cholesteryl esters (CEs) and ceramides (CERs). Covering these classes requires a multi-injection approach but data can be collected in short, fast gradients up 5 minutes run time per injection (for three injections). The three injections classes cover TAGs and SMs, CEs, CERs and DAGs and FFAs, PE/PCs and their lyso components (LPC/LPEs).

### MRM method preparation for each matrix

In order to be able to screen a particular matrix type for lipids, an MRM method is built for that particular matrix. The MRM method is built over time from experimental data, for example.

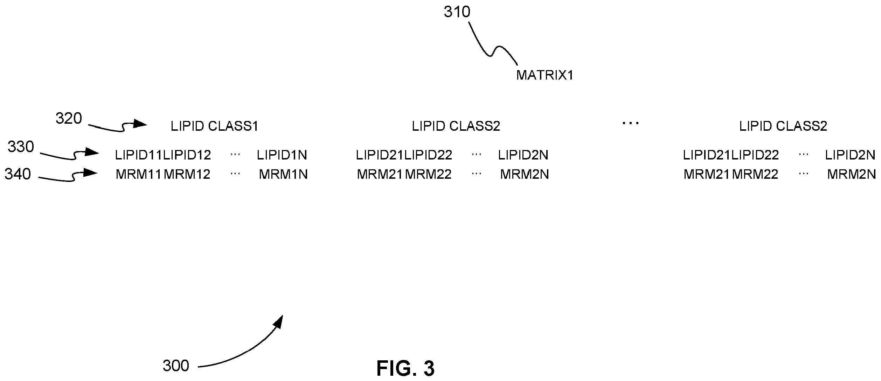

Figure 3 is a diagram 300 showing an initial hierarchy of an MRM method for identifying and quantitating lipids in a particular matrix, in accordance with various embodiments. Through experimentation, all the lipid classes 320 possible for a matrix 310 are identified. Matrix 310 is plasma, for example. Plasma can have on the order of 1200 different lipid species or molecules.

These 1200 different lipid species are grouped into lipid classes 320 based on similar chemical structure. The lipid classes for plasma can include, but are not limited to, phosphatidylcholine (PC), phosphatidylethanolamine (PE), lysophosphatidylcholine (LPC), lysophosphatidylethanolamine (LPE), triacylglycerol (TAG), diacylglycerol (DAG), cholesteryl esters (CE), free fatty acids (FFA), sphingomyelin (SM), ceramide (CER), hexosylceramide (HCer), lactosylceramide (LCer), and dihexosylceramide (DCer).

Each lipid class includes one or more lipids 330. Each lipid 330 is then identified and quantitated using one or more MRM transition 340. As described above, each MRM transition is a precursor ion mass-to-charge ratio (m/z) value and a product ion m/z value.

Also as described above, some lipids 330 may include MRM transition 340 precursor or product ion m/z values that are similar or the same as those of other lipids 330. This produces isobaric interferences. As a result, a DMS device is used to separate precursor ions during the analysis. A particular precursor ion is distinguished from other precursor ions by selecting a CoV DMS parameter value for the precursor ion. Since lipids of a particular class have a similar structure, a CoV value for a lipid class can be used to distinguish all the precursor ions of the class from lipids in other classes. In other words, a CoV value is experimentally determined for a lipid class.

Not all lipid classes320, however, include lipids 330 that have isobaric interferences with lipids 330 of classes. As a result, it is first determined what lipids 330 of matrix 310 have isobaric interferences. Then it is determined which lipid classes 320 include a lipid with an isobaric interference. Finally, a CoV value is determined for the lipid classes that have lipids with an isobaric interference. In other words, lipid classes 320 are further grouped as interference or classes with a CoV value, or as non-interference classes or classes without a CoV value.

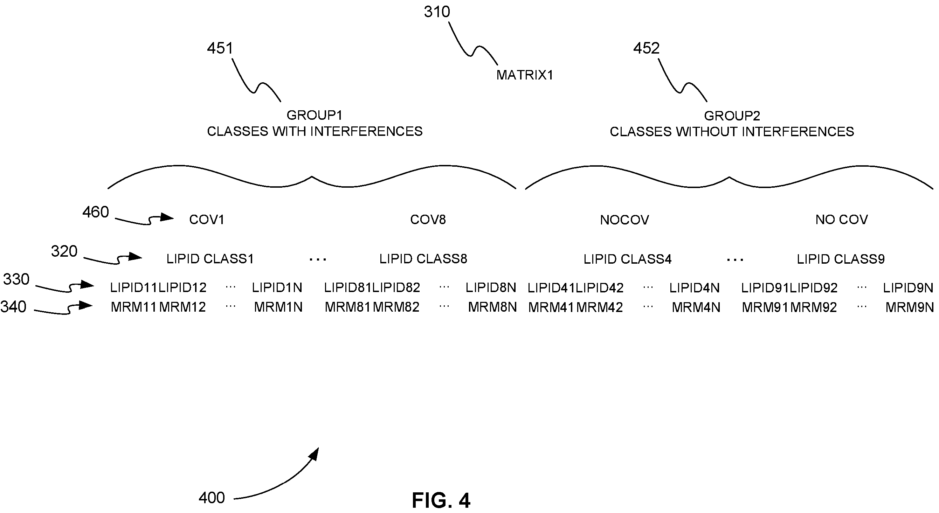

Figure 4 is a diagram 400 showing a hierarchy after grouping by isobaric interferences of an MRM method for identifying and quantitating lipids in a particular matrix, in accordance with various embodiments. Lipid classes 320 of matrix 310 are further grouped into one of two groups 451 and 452. Group 451 includes the lipid classes that include lipids with isobaric interferences, group 452 includes the lipid classes that do not include lipids with isobaric interferences. Further, for the lipid classes of group 451, DMS CoV parameter values 460 are experimentally found and assigned to each lipid class. No DMS CoV parameter values are assigned to the lipid classes of group 452.

Essentially, groups 451 and 452 allow the MS/MS to be performed with and without the DMS turned on, respectively. By separately analyzing these groups, the total analysis time using the instrument is decreased. In other words, analyzing the lipid classes of group 452 with the DMS acting as a pass through device decreases the overall analysis time.

In various embodiments, the analysis is further decreased by additionally ordering the lipid classes in group 451 according to the DMS CoV parameter value 460 of the lipid class. Because it takes time to change the CoV of the DMS and this time is dependent on the amount of the change, if the lipid classes are ordered according to increasing or decreasing CoV values, the data acquisition time can be reduced.

### System for quantitating lipids

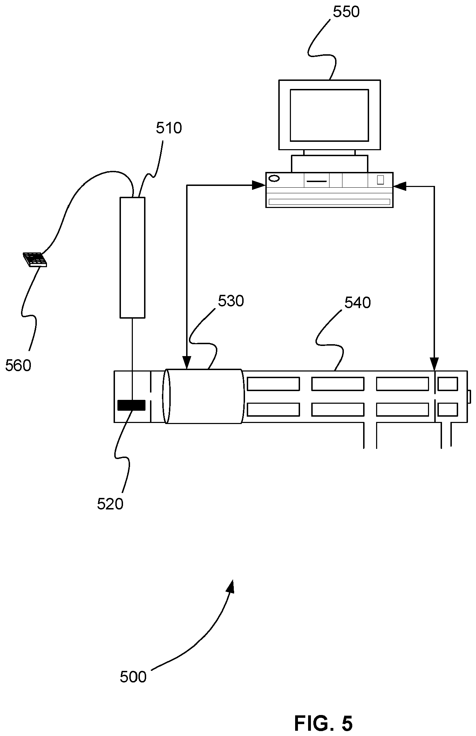

Figure 5 is a schematic diagram 500 showing a system for quantitating lipids of a matrix sample using a differential mobility spectrometry (DMS) device during a targeted multiple reaction monitoring (MRM) acquisition experiment, in accordance with various embodiments. The system of Figure 5 includes separation device 510, ion source 520, DMS device 530, tandem mass spectrometer 540, and processor 550.

Separation device 510 is configured to sequentially receive a first injection and a second injection of matrix sample 560. Separation device 510 is also configured to separate known lipid molecules of the matrix from the first injection and a second injection over time. Separation device 510 is, for example, a high performance liquid chromatography (HPLC) device. In various alternative embodiments, separation device 510 can perform one of a variety of separation techniques that include, but are not limited to, gas chromatography (GC), capillary electrophoresis (CE), or flow injection analysis (FIA).

In various embodiments, separation device 510 receives the first injection and the second injection through low-carryover tubing. Lipid molecules are sticking substances that easily adhere to surfaces. Low-carryover tubing prevents lipid molecules from adhering to tubing surfaces. One exemplary type of low-carryover tubing is PEEKsil™.

Before the experiment, the known lipid molecules of the matrix are grouped into lipid classes and the lipid classes are further grouped into a pass-through group and a mobility separation group. The pass-through group includes lipid classes without lipid molecules known to produce isobaric interferences with other lipid molecules. The mobility separation group includes lipid classes with lipid molecules known to produce isobaric interferences.

In various embodiments, lipid classes of the mobility separation group are further ordered according to increasing or decreasing CoV value to decrease the time needed to change CoV values of the DMS device during analysis of the second beam of ions.

Ion source 520 is configured to receive separated lipid molecules from separation device 510 for the first injection and the second injection. Ion source 520 is also configured to ionize the separated lipid molecules for the first injection and the second injection.

DMS device 530 is configured to receive a first beam of ions of the separated lipid molecules for the first injection and pass the first beam through without ion mobility separation. DMS device 530 is also configured to receive a second beam of ions of the separated lipid molecules for the second injection and sequentially mobility separate the second beam. The second beam of ions is sequentially separated by ion mobility according to CoV values experimentally predetermined for each lipid class of the mobility separation group. DMS device 530 can be a SelexION™ device, for example.

Tandem mass spectrometer 540 is configured to receive the first ion beam from the DMS device. Tandem mass spectrometer 540 is further configured to perform, for a first plurality of cycles, an MRM scan for at least one MRM transition for each lipid molecule of each lipid class of the pass-through group and store a first set of intensity values of each MRM scan for the first plurality of cycles.

Tandem mass spectrometer 540 is also configured to receive the second mobility separated ion beam from the DMS device. Tandem mass spectrometer 540 is further configured to perform, for a second plurality of cycles, an MRM scan for at least one MRM transition for each lipid molecule of each lipid class of the mobility separation group and store a second set of intensity values of each MRM scan for the second plurality of cycles. Each MRM transition of each lipid molecules is experimentally predetermined, for example.

In various embodiments, the first set of intensity values and the second set of intensity values are stored in a memory or data storage device. The data storage device can be a storage device of tandem mass spectrometer 540 or a separate storage device.

Tandem mass spectrometer 540 can include one or more physical mass filters and one or more physical mass analyzers. A mass analyzer of tandem mass spectrometer 540 can include, but is not limited to, a time-of-flight (TOF), a quadrupole, an ion trap, a linear ion trap, an orbitrap, or a Fourier transform mass analyzer.

In various embodiments, tandem mass spectrometer 540 further performs each MRM scan for each lipid molecule of the known lipid molecules of the matrix using a predetermined polarity for each lipid class.

Processor 550 is in communication with tandem mass spectrometer 540 and DMS device 530, for example. Processor 550 is configured or programmed to receive the first set of intensity values and the second set of intensity values, to receive MRM intensity and concentration values for known standards of the known lipid molecules of the matrix, and to quantitate each lipid molecule in matrix sample 560. Processor 550 quantitates each lipid molecule in matrix sample 560 by comparing the first set of intensity values and the second set of intensity values to the MRM intensity and concentration values for the known standards of the known lipid molecules of the matrix. For example, average intensities values are calculated for each MRM of each lipid molecule over the first plurality of cycles or the second plurality of cycles. The average intensities values are then compared to the MRM intensity and concentration values for the known standards of the known lipid molecules of the matrix.

In various embodiments, processor 550 receives the first set of intensity values, the second set of intensity values, and the MRM intensity and concentration values for known standards of the known lipid molecules of the matrix from a memory or data storage device. The data storage device can be a storage device of tandem mass spectrometer 540, a storage device of processor 550, or a separate storage device.

Processor 560 can be, but is not limited to, the system of Figure 1, a computer, microprocessor, or any device capable of sending and receiving control information and data to and from DMS device 530 and tandem mass spectrometer 540 and processing data.

In various embodiments, the quantitation is designed to accommodate differences in ionization efficiency of lipids classes as well as the differential fragmentation efficiency of lipids containing fatty acyl chains with different numbers of carbons and double bonds. Each lipid class has multiple internal standards that vary in chain length and degree of unsaturation, and have minimum or no interference with MRMs of target lipids. Target molecules are normalized by using the appropriate internal standard with the respective number of carbons and double bonds.

In various embodiments, a matrix for which lipid classes are experimentally predetermined can be an animal matrix. An animal matrix can include, but is not limited to, plasma, heart, liver, brain, muscle, whole blood, urine, or cells.

In various embodiments, a matrix for which lipid classes are experimentally predetermined can be a food matrix. A food matrix can include, but is not limited to, milk, edible oils, and meat.

In various embodiments, the lipid classes that the known lipid molecules of the matrix are grouped into include phosphatidylcholine (PC), phosphatidylethanolamine (PE), lysophosphatidylcholine (LPC), lysophosphatidylethanolamine (LPE), triacylglycerol (TAG), diacylglycerol (DAG), cholesteryl esters (CE), free fatty acids (FFA), sphingomyelin (SM), ceramide (CER), hexosylceramide (HCer), lactosylceramide (LCer), dihexosylceramide (DCer), phosphatidylserine (PS), phosphatidylglycerol (PG), phosphatidylinositol (PI), phosphatidic acid (PA), cardiolipin (CL), and oxidized fatty acid metabolites (eicosanoids).

In various embodiments, the matrix is plasma. The lipid classes that the known lipid molecules of plasma are grouped into include phosphatidylcholine (PC), phosphatidylethanolamine (PE), lysophosphatidylcholine (LPC), lysophosphatidylethanolamine (LPE), triacylglycerol (TAG), diacylglycerol (DAG), cholesteryl esters (CE), free fatty acids (FFA), sphingomyelin (SM), ceramide (CER), hexosylceramide (HCer), lactosylceramide (LCer), and dihexosylceramide (DCer).

### Method for quantitating lipids

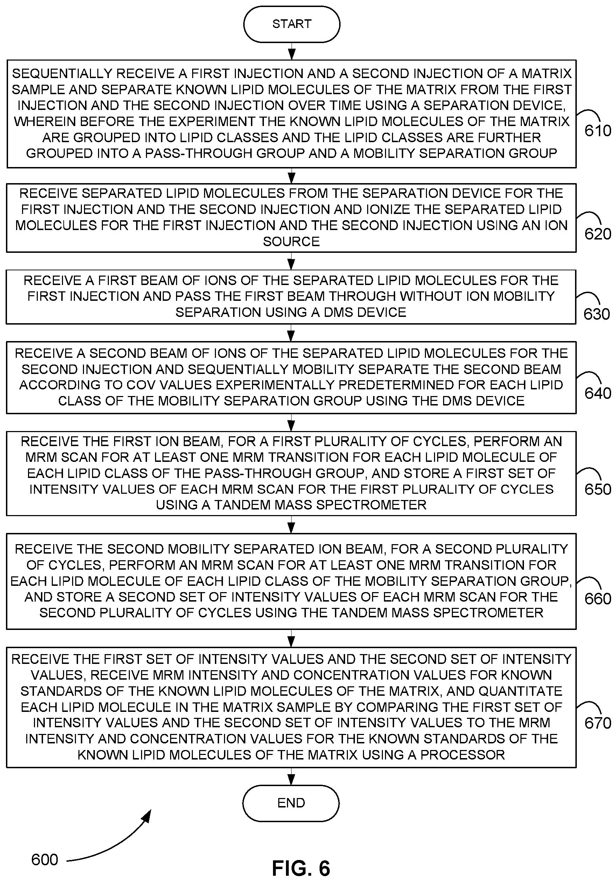

Figure 6 is a flowchart 600 showing a method for quantitating lipids of a matrix sample using a DMS device during a targeted MRM acquisition experiment, in accordance with various embodiments.

In step 610 of the method of Figure 6, a first injection and a second injection of a matrix sample are sequentially received and known lipid molecules of the matrix are separated from the first injection and the second injection over time using a separation device. Before the experiment, the known lipid molecules of the matrix are grouped into lipid classes. The lipid classes are further grouped into a pass-through group that includes lipid classes without lipid molecules known to produce isobaric interferences with other lipid molecules and a mobility separation group that includes lipid classes with lipid molecules known to produce isobaric interferences.

In step 620, separated lipid molecules are received from the separation device for the first injection and the second injection and the separated lipid molecules are ionized for the first injection and the second injection using an ion source.

In step 630, a first beam of ions of the separated lipid molecules for the first injection is received and the first beam is passed through without ion mobility separation using a DMS device.

In step 640, a second beam of ions of the separated lipid molecules for the second injection is received and the second beam is sequentially mobility separated according to CoV values experimentally predetermined for each lipid class of the mobility separation group using the DMS device.

In step 650, the first ion beam is received from the DMS device, for a first plurality of cycles, an MRM scan is performed for at least one MRM transition for each lipid molecule of each lipid class of the pass-through group, and a first set of intensity values of each MRM scan for the first plurality of cycles is stored using a tandem mass spectrometer.

In step 660, the second mobility separated ion beam is received from the DMS device, for a second plurality of cycles, an MRM scan is performed for at least one MRM transition for each lipid molecule of each lipid class of the mobility separation group, and a second set of intensity values of each MRM scan for the second plurality of cycles is stored using the tandem mass spectrometer. Each MRM transition of each lipid molecules is experimentally predetermined; for example.

In step 670, the first set of intensity values and the second set of intensity values are received, MRM intensity and concentration values for known standards of the known lipid molecules of the matrix are received, and each lipid molecule in the matrix sample is quantitated using a processor. Each lipid molecule in the matrix sample is quantitated by comparing the first set of intensity values and the second set of intensity values to the MRM intensity and concentration values for the known standards of the known lipid molecules of the matrix.

While the present teachings are described in conjunction with various embodiments, it is not intended that the present teachings be limited to such embodiments. On the contrary, the present teachings encompass various alternatives, modifications, and equivalents, as will be appreciated by those of skill in the art.

Further, in describing various embodiments, the specification may have presented a method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process should not be limited to the performance of their steps in the order written, and one skilled in the art can readily appreciate that the sequences may be varied within the scope of the invention as defined by the appended claims.

## Claims

1. A system for quantitating lipids of a matrix sample using a differential mobility spectrometry (DMS) device during a targeted multiple reaction monitoring (MRM) acquisition experiment, comprising:
a separation device (510) configured to receive sequentially a first injection and a second injection of a matrix sample and to separate known lipid molecules of the matrix from the first injection and the second injection over time, wherein before the experiment the known lipid molecules of the matrix are grouped into lipid classes and the lipid classes are further grouped into a pass-through group (452) that includes lipid classes without lipid molecules known to produce isobaric interferences with other lipid molecules and a mobility separation group (451) that includes lipid classes with lipid molecules known to produce isobaric interferences;
an ion source (520) configured to receive separated lipid molecules from the separation device for the first injection and the second injection and to ionize the separated lipid molecules for the first injection and the second injection;
a DMS device (530) configured to receive a first beam of ions of the separated lipid molecules for the first injection and pass the first beam through without ion mobility separation, and to receive a second beam of ions of the separated lipid molecules for the second injection and sequentially mobility separate the second beam according to compensation voltage (CoV) values experimentally predetermined for each lipid class of the mobility separation group;
a tandem mass spectrometer (540) configured to receive the first ion beam from the DMS device, for a first plurality of cycles, perform an MRM scan for at least one MRM transition for each lipid molecule of each lipid class of the pass-through group (452), and store a first set of intensity values of each MRM scan for the first plurality of cycles, and to receive the second mobility separated ion beam from the DMS device, for a second plurality of cycles, perform an MRM scan for at least one MRM transition for each lipid molecule of each lipid class of the mobility separation group (451), and store a second set of intensity values of each MRM scan for the second plurality of cycles, wherein each MRM transition of each lipid molecules is experimentally predetermined;
a processor (550) in communication with the mass spectrometer (540) and the DMS device (530) configured to receive the first set of intensity values and the second set of intensity values, to receive MRM intensity and concentration values for known standards of the known lipid molecules of the matrix, and to quantitate each lipid molecule in the matrix sample by comparing the first set of intensity values and the second set of intensity values to the MRM intensity and concentration values for the known standards of the known lipid molecules of the matrix.

2. The system of claim 1, wherein the separation device comprises a high performance liquid chromatography (HPLC) device.

3. The system of claim 1, wherein the separation device receives the first injection and the second injection through low-carryover tubing.

4. The system of claim 1, wherein the tandem mass spectrometer further performs each MRM scan for each lipid molecule of the known lipid molecules of the matrix using a predetermined polarity for each lipid class.

5. The system of claim 1, wherein lipid classes of the mobility separation group are further ordered according to increasing CoV value to decrease the time needed to change CoV values of the DMS device during analysis of the second beam of ions.

6. The system of claim 1, wherein lipid classes of the mobility separation group are further ordered according to decreasing CoV value to decrease the time needed to change CoV values of the DMS device during analysis of the second beam of ions.

7. The system of claim 1, wherein the matrix comprises one of plasma, heart, liver, brain, muscle, whole blood, urine, and cells.

8. The system of claim 1, wherein the matrix comprises one of milk, edible oils, and meat.

9. The system of claim 1, wherein the lipid classes that the known lipid molecules of the matrix are grouped into comprise phosphatidylcholine (PC), phosphatidylethanolamine (PE), lysophosphatidylcholine (LPC), lysophosphatidylethanolamine (LPE), triacylglycerol (TAG), diacylglycerol (DAG), cholesteryl esters (CE), free fatty acids (FFA), sphingomyelin (SM), ceramide (CER), hexosylceramide (HCer), lactosylceramide (LCer), dihexosylceramide (DCer), phosphatidylserine (PS), phosphatidylglycerol (PG), phosphatidylinositol (PI), phosphatidic acid (PA), cardiolipin (CL), and oxidized fatty acid metabolites (eicosanoids).

10. The system of claim 1, wherein the matrix comprises plasma and the lipid classes that the known lipid molecules of plasma are grouped into comprise phosphatidylcholine (PC), phosphatidylethanolamine (PE), lysophosphatidylcholine (LPC), lysophosphatidylethanolamine (LPE), triacylglycerol (TAG), diacylglycerol (DAG), cholesteryl esters (CE), free fatty acids (FFA), sphingomyelin (SM), ceramide (CER), hexosylceramide (HCer), lactosylceramide (LCer), and dihexosylceramide (DCer).

11. A method for quantitating lipids of a matrix sample using a differential mobility spectrometry (DMS) device during a targeted multiple reaction monitoring (MRM) acquisition experiment, comprising:
sequentially receiving (610) a first injection and a second injection of a matrix sample and separating known lipid molecules of the matrix from the first injection and the second injection over time using a separation device, wherein before the experiment the known lipid molecules of the matrix are grouped into lipid classes and the lipid classes are further grouped into a pass-through (452) group that includes lipid classes without lipid molecules known to produce isobaric interferences with other lipid molecules and a mobility separation group (451) that includes lipid classes with lipid molecules known to produce isobaric interferences;
receiving (620) separated lipid molecules from the separation device for the first injection and the second injection and ionizing the separated lipid molecules for the first injection and the second injection using an ion source;
receiving (630) a first beam of ions of the separated lipid molecules for the first injection and passing the first beam through without ion mobility separation using a DMS device;
receiving (640) a second beam of ions of the separated lipid molecules for the second injection and sequentially mobility separating the second beam according to compensation voltage (CoV) values experimentally predetermined for each lipid class of the mobility separation group using the DMS device;
receiving (650) the first ion beam from the DMS device, for a first plurality of cycles, performing an MRM scan for at least one MRM transition for each lipid molecule of each lipid class of the pass-through group, and storing a first set of intensity values of each MRM scan for the first plurality of cycles using a tandem mass spectrometer;
receiving (660) the second mobility separated ion beam from the DMS device, for a second plurality of cycles, performing an MRM scan for at least one MRM transition for each lipid molecule of each lipid class of the mobility separation group, and storing a second set of intensity values of each MRM scan for the second plurality of cycles using the tandem mass spectrometer, wherein each MRM transition of each lipid molecules is experimentally predetermined; and
receiving (670) the first set of intensity values and the second set of intensity values, receiving MRM intensity and concentration values for known standards of the known lipid molecules of the matrix, and quantitating each lipid molecule in the matrix sample by comparing the first set of intensity values and the second set of intensity values to the MRM intensity and concentration values for the known standards of the known lipid molecules of the matrix using a processor.

12. The method of claim 11, wherein separating known lipid molecules of the matrix from the first injection and the second injection over time comprises performing high performance liquid chromatography (HPLC).

13. The method of claim 11, wherein performing an MRM scan for at least one MRM transition for each lipid molecule of each lipid class further comprises using a predetermined polarity for each lipid class.

14. The method of claim 11, wherein lipid classes of the mobility separation group are further ordered according to decreasing CoV value to decrease the time needed to change CoV values of the DMS device during analysis of the second beam of ions.

15. The method of claim 11, wherein lipid classes of the mobility separation group are further ordered according to increasing CoV value to decrease the time needed to change CoV values of the DMS device during analysis of the second beam of ions.

## Patentansprüche

1. System zur Quantifizierung von Lipiden einer Matrixprobe unter Verwendung einer Differential-Mobilitäts-Spektrometrie (DMS)-Vorrichtung während eines gezielten Erfassungsexperiments zur Mehrfachreaktionsüberwachung (MRM), umfassend:
eine Trennvorrichtung (510), die konfiguriert ist, um sequenziell eine erste Injektion und eine zweite Injektion einer Matrixprobe zu empfangen und bekannte Lipidmoleküle der Matrix von der ersten Injektion und der zweiten Injektion über einen bestimmten Zeitraum hinweg abzutrennen, wobei vor dem Experiment die bekannten Lipidmoleküle der Matrix in Lipidklassen gruppiert werden und die Lipidklassen weiter gruppiert werden in eine Durchlassgruppe (452), die Lipidklassen ohne Lipidmoleküle umfasst, von denen bekannt ist, dass sie isobare Interferenzen mit anderen Lipidmolekülen erzeugen, und eine Mobilitätstrennungsgruppe (451), die Lipidklassen mit Lipidmolekülen umfasst, von denen bekannt ist, dass sie isobare Interferenzen erzeugen;
eine Ionenquelle (520), die konfiguriert ist, um abgetrennte Lipidmoleküle von der Trennvorrichtung für die erste Injektion und die zweite Injektion zu empfangen und die abgetrennten Lipidmoleküle für die erste Injektion und die zweite Injektion zu ionisieren;
eine DMS-Vorrichtung (530), die konfiguriert ist, um einen ersten Ionenstrahl der abgetrennten Lipidmoleküle für die erste Injektion zu empfangen und den ersten Strahl ohne Ionenmobilitätstrennung durchzulassen und um einen zweiten Ionenstrahl der abgetrennten Lipidmoleküle für die zweite Injektion zu empfangen und für den zweiten Strahl in Übereinstimmung mit Kompensationsspannungswerten (CoV), die experimentell für jede Lipidklasse der Mobilitätstrennungsgruppe vorbestimmt sind, sequenziell eine Mobilitätstrennung durchzuführen;
ein Tandem-Massenspektrometer (540), das konfiguriert ist, um den ersten Ionenstrahl von der DMS-Vorrichtung für eine erste Vielzahl von Zyklen zu empfangen, einen MRM-Scan für mindestens einen MRM-Übergang für jedes Lipidmolekül jeder Lipidklasse der Durchgangsgruppe (452) durchzuführen und einen ersten Satz von Intensitätswerten jedes MRM-Scans für die erste Vielzahl von Zyklen zu speichern und um den zweiten mobilitätsgetrennten Ionenstrahl von der DMS-Vorrichtung für eine zweite Vielzahl von Zyklen zu empfangen, einen MRM-Scan für mindestens einen MRM-Übergang für jedes Lipidmolekül jeder Lipidklasse der Mobilitätstrennungsgruppe (451) durchzuführen und einen zweiten Satz von Intensitätswerten jedes MRM-Scans für die zweite Vielzahl von Zyklen zu speichern, wobei jeder MRM-Übergang jedes Lipidmoleküls experimentell vorbestimmt ist;
einen Prozessor (550) in Kommunikation mit dem Massenspektrometer (540) und der DMS-Vorrichtung (530), der konfiguriert ist, um den ersten Satz von Intensitätswerten und den zweiten Satz von Intensitätswerten zu empfangen, um MRM-Intensitäts- und Konzentrationswerte für bekannte Standards der bekannten Lipidmoleküle der Matrix zu empfangen und jedes Lipidmolekül in der Matrixprobe durch Vergleichen des ersten Satzes von Intensitätswerten und des zweiten Satzes von Intensitätswerten mit den MRM-Intensitäts- und Konzentrationswerten für die bekannten Standards der bekannten Lipidmoleküle der Matrix zu quantifizieren.

2. System nach Anspruch 1, wobei die Trennvorrichtung eine Hochleistungsflüssigkeitschromatographie (HPLC)-Vorrichtung umfasst.

3. System nach Anspruch 1, wobei die Trennvorrichtung die erste Injektion und die zweite Injektion durch eine Leitung mit geringem Übertrag empfängt.

4. System nach Anspruch 1, wobei das Tandem-Massenspektrometer ferner jeden MRM-Scan für jedes Lipidmolekül der bekannten Lipidmoleküle der Matrix unter Verwendung einer vorbestimmten Polarität für jede Lipidklasse durchführt.

5. System nach Anspruch 1, wobei die Lipidklassen der Mobilitätstrennungsgruppe ferner nach zunehmendem CoV-Wert angeordnet sind, um die Zeit zu verringern, die zum Ändern der CoV-Werte der DMS-Vorrichtung während der Analyse des zweiten Ionenstrahls benötigt wird.

6. System nach Anspruch 1, wobei die Lipidklassen der Mobilitätstrennungsgruppe ferner nach abnehmendem CoV-Wert angeordnet sind, um die Zeit zu verringern, die zum Ändern der CoV-Werte der DMS-Vorrichtung während der Analyse des zweiten Ionenstrahls benötigt wird.

7. System nach Anspruch 1, wobei die Matrix eines von Plasma, Herz, Leber, Gehirn, Muskel, Vollblut, Urin und Zellen umfasst.

8. System nach Anspruch 1, wobei die Matrix eines von Milch, Speiseölen und Fleisch umfasst.

9. System nach Anspruch 1, wobei die Lipidklassen, in die die bekannten Lipidmoleküle der Matrix gruppiert sind, Phosphatidylcholin (PC), Phosphatidylethanolamin (PE), Lysophosphatidylcholin (LPC), Lysophosphatidylethanolamin (LPE), Triacylglycerin (TAG), Diacylglycerin (DAG), Cholesterylester (CE), freie Fettsäuren (FFA), Sphingomyelin (SM), Ceramid (CER), Hexosylceramid (HCer), Lactosylceramid (LCer), Dihexosylceramid (DCer), Phosphatidylserin (PS), Phosphatidylglycerin (PG), Phosphatidylinositol (PI), Phosphatidsäure (PA), Cardiolipin (CL) und oxidierte Fettsäuremetabolite (Eicosanoide) umfassen.

10. System nach Anspruch 1, wobei die Matrix Plasma umfasst und die Lipidklassen, in die die bekannten Lipidmoleküle des Plasmas gruppiert sind, Phosphatidylcholin (PC), Phosphatidylethanolamin (PE), Lysophosphatidylcholin (LPC), Lysophosphatidylethanolamin (LPE), Triacylglycerin (TAG), Diacylglycerin (DAG), Cholesterylester (CE), freie Fettsäuren (FFA), Sphingomyelin (SM), Ceramid (CER), Hexosylceramid (HCer), Lactosylceramid (LCer) und Dihexosylceramid (DCer) umfassen.

11. Verfahren zur Quantifizierung von Lipiden einer Matrixprobe unter Verwendung einer Differential-Mobilitäts-Spektrometrie (DMS)-Vorrichtung während eines gezielten Erfassungsexperiments zur Mehrfachreaktionsüberwachung (MRM), umfassend:
sequenzielles Empfangen (610) einer ersten Injektion und einer zweiten Injektion einer Matrixprobe und Abtrennen bekannter Lipidmoleküle der Matrix von der ersten Injektion und der zweiten Injektion über einen bestimmten Zeitraum hinweg unter Verwendung einer Trennvorrichtung, wobei vor dem Experiment die bekannten Lipidmoleküle der Matrix in Lipidklassen gruppiert werden und die Lipidklassen weiter gruppiert werden in eine Durchlassgruppe (452), die Lipidklassen ohne Lipidmoleküle umfasst, von denen bekannt ist, dass sie isobare Interferenzen mit anderen Lipidmolekülen erzeugen, und eine Mobilitätstrennungsgruppe (451), die Lipidklassen mit Lipidmolekülen umfasst, von denen bekannt ist, dass sie isobare Interferenzen erzeugen;
Empfangen (620) abgetrennter Lipidmoleküle von der Trennvorrichtung für die erste Injektion und die zweite Injektion und Ionisieren der abgetrennten Lipidmoleküle für die erste Injektion und die zweite Injektion unter Verwendung einer Ionenquelle;
Empfangen (630) eines ersten Ionenstrahls der abgetrennten Lipidmoleküle für die erste Injektion und Durchlassen des ersten Strahls ohne Ionenmobilitätstrennung unter Verwendung einer DMS-Vorrichtung;
Empfangen (640) eines zweiten Ionenstrahls der abgetrennten Lipidmoleküle für die zweite Injektion und sequenzielle Mobilitätstrennung des zweiten Strahls in Übereinstimmung mit Kompensationsspannungswerten (CoV), die experimentell für jede Lipidklasse der Mobilitätstrennungsgruppe vorbestimmt sind, unter Verwendung der DMS-Vorrichtung;
Empfangen (650) des ersten Ionenstrahls von der DMS-Vorrichtung für eine erste Vielzahl von Zyklen, Durchführen eines MRM-Scans für mindestens einen MRM-Übergang für jedes Lipidmolekül jeder Lipidklasse der Durchlassgruppe und Speichern eines ersten Satzes von Intensitätswerten jedes MRM-Scans für die erste Vielzahl von Zyklen unter Verwendung eines Tandem-Massenspektrometers;
Empfangen (660) des zweiten mobilitätsgetrennten Ionenstrahls von der DMS-Vorrichtung für eine zweite Vielzahl von Zyklen, Durchführen eines MRM-Scans für mindestens einen MRM-Übergang für jedes Lipidmolekül jeder Lipidklasse der Mobilitätstrennungsgruppe und Speichern eines zweiten Satzes von Intensitätswerten jedes MRM-Scans für die zweite Vielzahl von Zyklen unter Verwendung des Tandem-Massenspektrometers, wobei jeder MRM-Übergang jedes Lipidmoleküls experimentell vorbestimmt ist; und
Empfangen (670) des ersten Satzes von Intensitätswerten und des zweiten Satzes von Intensitätswerten, Empfangen von MRM-Intensitäts- und Konzentrationswerten für bekannte Standards der bekannten Lipidmoleküle der Matrix und Quantifizieren jedes Lipidmoleküls in der Matrixprobe durch Vergleichen des ersten Satzes von Intensitätswerten und des zweiten Satzes von Intensitätswerten mit den MRM-Intensitäts- und Konzentrationswerten für die bekannten Standards der bekannten Lipidmoleküle der Matrix unter Verwendung eines Prozessors.

12. Verfahren nach Anspruch 11, wobei das Abtrennen bekannter Lipidmoleküle der Matrix von der ersten Injektion und der zweiten Injektion über einen bestimmten Zeitraum hinweg die Durchführung einer Hochleistungsflüssigkeitschromatographie (HPLC) umfasst.

13. Verfahren nach Anspruch 11, wobei die Durchführung eines MRM-Scans für mindestens einen MRM-Übergang für jedes Lipidmolekül jeder Lipidklasse ferner die Verwendung einer vorbestimmten Polarität für jede Lipidklasse umfasst.

14. Verfahren nach Anspruch 11, wobei die Lipidklassen der Mobilitätstrennungsgruppe ferner nach abnehmendem CoV-Wert angeordnet sind, um die Zeit zu verringern, die zum Ändern der CoV-Werte der DMS-Vorrichtung während der Analyse des zweiten Ionenstrahls benötigt wird.

15. Verfahren nach Anspruch 11, wobei die Lipidklassen der Mobilitätstrennungsgruppe ferner nach zunehmendem CoV-Wert angeordnet sind, um die Zeit zu verringern, die zum Ändern der CoV-Werte der DMS-Vorrichtung während der Analyse des zweiten Ionenstrahls benötigt wird.

## Revendications

1. Système pour quantifier les lipides d'un échantillon de matrice en utilisant un dispositif de spectrométrie de mobilité différentielle (DMS) pendant une expérience d'acquisition de surveillance de réactions multiples ciblée (MRM), comprenant :
un dispositif de séparation (510) configuré pour recevoir séquentiellement une première injection et une deuxième injection d'un échantillon de matrice et pour séparer les molécules lipidiques connues de la matrice à partir de la première injection et de la deuxième injection dans le temps, dans lequel avant l'expérience les molécules lipidiques connues de la matrice sont regroupées en classes lipidiques et les classes lipidiques sont en outre regroupées en un groupe de passage (452) qui comprend des classes lipidiques sans molécules lipidiques connues pour produire des interférences isobares avec d'autres molécules lipidiques et un groupe de séparation de mobilité (451) qui comprend des classes lipidiques avec des molécules lipidiques connues pour produire des interférences isobariques ;
une source ionique (520) configurée pour recevoir des molécules lipidiques séparées à partir du dispositif de séparation pour la première injection et la deuxième injection et pour ioniser les molécules lipidiques séparées pour la première injection et la deuxième injection ;
un dispositif DMS (530) configuré pour recevoir un premier faisceau ionique des molécules lipidiques séparées pour la première injection et faire passer le premier faisceau sans séparation par mobilité ionique, et pour recevoir un deuxième faisceau ionique des molécules lipidiques séparées pour la deuxième injection et séparer par mobilité séquentiellement le deuxième faisceau selon des valeurs de tension de compensation (CoV) prédéterminées expérimentalement pour chaque classe lipidique du groupe de séparation par mobilité ;
un spectromètre de masse en tandem (540) configuré pour recevoir le premier faisceau ionique à partir du dispositif DMS, pour une première pluralité de cycles, pour effectuer un balayage MRM pour au moins une transition MRM pour chaque molécule lipidique de chaque classe lipidique du groupe de passage (452), et pour stocker un premier ensemble de valeurs d'intensité de chaque balayage MRM pour la première pluralité de cycles, et pour recevoir le deuxième faisceau ionique séparé par mobilité à partir du dispositif DMS, pour une deuxième pluralité de cycles, pour effectuer un balayage MRM pour au moins une transition MRM pour chaque molécule lipidique de chaque classe lipidique du groupe de séparation de mobilité (451), et stocker un deuxième ensemble de valeurs d'intensité de chaque balayage MRM pour la deuxième pluralité de cycles, dans lequel chaque transition MRM de chaque molécule lipidique est expérimentalement prédéterminée ;
un processeur (550) en communication avec le spectromètre de masse (540) et le dispositif DMS (530) configuré pour recevoir le premier ensemble de valeurs d'intensité et le deuxième ensemble de valeurs d'intensité, pour recevoir des valeurs d'intensité et de concentration MRM pour des normes connues des molécules lipidiques connues de la matrice, et pour quantifier chaque molécule lipidique dans l'échantillon de matrice en comparant le premier ensemble de valeurs d'intensité et le deuxième ensemble de valeurs d'intensité aux valeurs d'intensité et de concentration MRM pour les normes connues des molécules lipidiques connues de la matrice.

2. Système selon la revendication 1, dans lequel le dispositif de séparation comprend un dispositif de chromatographie liquide à haute performance (HPLC).

3. Système selon la revendication 1, dans lequel le dispositif de séparation reçoit la première injection et la deuxième injection à travers une tubulure à faible transfert.

4. Système selon la revendication 1, dans lequel le spectromètre de masse en tandem effectue en outre chaque balayage MRM pour chaque molécule lipidique des molécules lipidiques connues de la matrice en utilisant une polarité prédéterminée pour chaque classe lipidique.

5. Système selon la revendication 1, dans lequel les classes lipidiques du groupe de séparation de mobilité sont en outre ordonnées en fonction de l'augmentation de la valeur CoV pour diminuer le temps nécessaire pour changer les valeurs CoV du dispositif DMS pendant l'analyse du deuxième faisceau ionique.

6. Système selon la revendication 1, dans lequel les classes lipidiques du groupe de séparation de mobilité sont en outre ordonnées en fonction de la valeur CoV décroissante pour diminuer le temps nécessaire pour changer les valeurs CoV du dispositif DMS pendant l'analyse du deuxième faisceau ionique.

7. Système selon la revendication 1, dans lequel la matrice comprend l'un parmi le plasma, le cœur, le foie, le cerveau, les muscles, le sang total, l'urine et les cellules.

8. Système selon la revendication 1, dans lequel la matrice comprend l'un parmi le lait, les huiles comestibles et la viande.

9. Système selon la revendication 1, dans lequel les classes lipidiques dans lesquelles les molécules lipidiques connues de la matrice sont regroupées comprennent la phosphatidylcholine (PC), la phosphatidyléthanolamine (PE), la lysophosphatidylcholine (LPC), la lysophosphatidyléthanolamine (LPE), le triacylglycérol (TAG), le diacylglycérol (DAG), les esters de cholestérol (CE), les acides gras libres (FFA), la sphingomyéline (SM), le céramide (CER), l'hexosylcéramide (HCer), le lactosylcéramide (LCer), le dihexosylcéramide (DCer), la phosphatidylsérine (PS), le phosphatidylglycérol (PG), le phosphatidylinositol (PI), l'acide phosphatidique (PA), la cardiolipine (CL) et les métabolites d'acides gras oxydés (eicosanoïdes).

10. Système selon la revendication 1, dans lequel la matrice comprend du plasma et les classes lipidiques dans lesquelles les molécules lipidiques connues du plasma sont regroupées comprennent la phosphatidylcholine (PC), la phosphatidyléthanolamine (PE), la lysophosphatidylcholine (LPC), la lysophosphatidyléthanolamine (LPE), le triacylglycérol (TAG), le diacylglycérol (DAG), les esters de cholestéryle (CE), les acides gras libres (FFA), la sphingomyéline (SM), le céramide (CER), l'hexosylcéramide (HCer), le lactosylcéramide (LCer) et le dihexosylcéramide (DCer).

11. Procédé de quantification des lipides d'un échantillon de matrice utilisant un dispositif de spectrométrie de mobilité différentielle (DMS) pendant une expérience d'acquisition de surveillance de réactions multiples ciblée (MRM), comprenant :
la réception séquentielle (610) d'une première injection et d'une deuxième injection d'un échantillon de matrice et la séparation des molécules lipidiques connues de la matrice de la première injection et de la deuxième injection dans le temps en utilisant un dispositif de séparation, dans lequel avant l'expérience les molécules lipidiques connues de la matrice sont regroupées en classes lipidiques et les classes lipidiques sont en outre regroupées en un groupe de passage (452) qui comprend des classes lipidiques sans molécules lipidiques connues pour produire des interférences isobariques avec d'autres molécules lipidiques et un groupe de séparation de mobilité (451) qui comprend des classes lipidiques avec des molécules lipidiques connues pour produire des interférences isobariques ;
la réception (620) des molécules lipidiques séparées à partir du dispositif de séparation pour la première injection et la deuxième injection et l'ionisation des molécules lipidiques séparées pour la première injection et la deuxième injection en utilisant une source ionique ;
la réception (630) d'un premier faisceau ionique des molécules lipidiques séparées pour la première injection et le passage du premier faisceau sans séparation par mobilité ionique en utilisant un dispositif DMS ;
la réception (640) d'un deuxième faisceau ionique des molécules lipidiques séparées pour la deuxième injection et la séparation séquentielle par mobilité du deuxième faisceau selon des valeurs de tension de compensation (CoV) prédéterminées expérimentalement pour chaque classe lipidique du groupe de séparation de mobilité en utilisant le dispositif DMS ;
la réception (650) du premier faisceau ionique à partir du dispositif DMS, pour une première pluralité de cycles, l'exécution d'un balayage MRM pour au moins une transition MRM pour chaque molécule lipidique de chaque classe lipidique du groupe de passage, et le stockage d'un premier ensemble des valeurs d'intensité de chaque balayage MRM pour la première pluralité de cycles en utilisant un spectromètre de masse en tandem ;
la réception (660) du deuxième faisceau ionique séparé par mobilité à partir du dispositif DMS, pour une deuxième pluralité de cycles, l'exécution d'un balayage MRM pour au moins une transition MRM pour chaque molécule lipidique de chaque classe lipidique du groupe de séparation de mobilité, et le stockage d'un deuxième ensemble de valeurs d'intensité de chaque balayage MRM pour la deuxième pluralité de cycles en utilisant le spectromètre de masse en tandem, dans lequel chaque transition MRM de chaque molécule lipidique est prédéterminée expérimentalement ; et
la réception (670) du premier ensemble de valeurs d'intensité et du deuxième ensemble de valeurs d'intensité, la réception des valeurs d'intensité et de concentration MRM pour des normes connues des molécules lipidiques connues de la matrice, et la quantification de chaque molécule lipidique dans l'échantillon de matrice en comparant le premier ensemble de valeurs d'intensité et le deuxième ensemble de valeurs d'intensité aux valeurs d'intensité et de concentration MRM pour les normes connues des molécules lipidiques connues de la matrice en utilisant un processeur.

12. Procédé selon la revendication 11, dans lequel la séparation des molécules lipidiques connues de la matrice de la première injection et de la deuxième injection dans le temps comprend la réalisation d'une chromatographie liquide à haute performance (HPLC).

13. Procédé selon la revendication 11, dans lequel la réalisation d'un balayage MRM pour au moins une transition MRM pour chaque molécule lipidique de chaque classe lipidique comprend en outre l'utilisation d'une polarité prédéterminée pour chaque classe lipidique.

14. Procédé selon la revendication 11, dans lequel les classes lipidiques du groupe de séparation de mobilité sont en outre ordonnées en fonction de la valeur CoV décroissante pour diminuer le temps nécessaire pour changer les valeurs CoV du dispositif DMS pendant l'analyse du deuxième faisceau ionique.

15. Procédé selon la revendication 11, dans lequel les classes lipidiques du groupe de séparation de mobilité sont en outre ordonnées en fonction de la valeur CoV croissante pour diminuer le temps nécessaire pour changer les valeurs CoV du dispositif DMS pendant l'analyse du deuxième faisceau ionique.
